# EUROPEAN PATENT SPECIFICATION

(11) **EP 3 469 916 B1**
(45) Date of publication and mention of the grant of the patent: **15.04.2020**
(21) Application number: 17001704.0
(22) Date of filing: 16.10.2017
(51) Int. Cl.: A23G 9/36, A23G 9/38, A61K 31/07, A61K 31/593, A61K 38/01

(54) **ORAL DELIVERY SYSTEM FOR COLLAGEN PEPTIDES**
ORALES VERABREICHUNGSSYSTEM FÜR KOLLAGENPEPTIDE
SYSTÈME D'ADMINISTRATION PAR VOIE ORALE DE PEPTIDES DE COLLAGÈNE

(43) Date of publication of application: 17.04.2019
(73) Proprietor: Derin, Anatoly, Hallandale Beach, Florida 33009 (US)
(72) Inventor: Derin, Anatoly, Hallandale Beach, Florida 33009 (US)
(74) Representative: Garavelli, Paolo

(56) References cited:
- EP-A1- 2 088 157
- EP-A1- 2 426 138
- LIYING LI ET AL: "Effect of Porcine Collagen Peptides on the Rheological and Sensory Properties of Ice Cream", KOREAN JOURNAL FOR FOOD SCIENCE OF ANIMAL RESOURCES, vol. 35, no. 2, 30 April 2015 (2015-04-30) , pages 156-163, XP055216083, ISSN: 1225-8563, DOI: 10.5851/kosfa.2015.35.2.156
- My Nuyen: "Coconut Berry Ice-cream sandwiches with Collagen Peptides", , 13 August 2017 (2017-08-13), XP002779313, Retrieved from the Internet: URL:https://myhealthydish.com/desserts/coc onut-berry-ice-cream-sandwiches-with-colla gen-peptides/ [retrieved on 2018-03-20]

## Description

The present invention provides a process for preparing an oral delivery system of collagen peptide, suitable for administration to both human and non-human animals, by means of frozen confections.

This delivery system may additionally contain other bioactive ingredients such as nutraceuticals, botanicals, and vitamins. The delivery system comprises a frozen dessert, collagen peptide (hydrolysate) and one or more bioactive ingredients which are substantially uniformly and completely dispersed and in which degradation of collagen peptide and other bioactives is minimized or eliminated. The invention also provides methods of preparing and using the delivery system.

The present invention provides a very unique and unconventional solution in the constant search for new and unconventional solutions, methods and processes to provide to consumers functional products with well-studied and recorded anti-aging effects. The present invention presents a functional frozen dessert which delivers an anti-aging effect to consumers. This unique delivery system intertwines and connects the beauty and health industries with frozen confections and food supplements industries. This delivery system expands the world of health and beauty in a new and unique format of a functional frozen dessert. The benefits of collagen peptides have been clinically studied and well documented. The studies has shown the collagen peptide supplementation significantly increases skin hydration and collagen density in the dermis. It significantly induces collagen as well as glycosaminoglycan production, offering an effective mechanism for reducing the signs of aging.

Although anyone can benefit from a daily moderate supplementation of collagen peptides in the diet, the targeted demographic group is primarily thought to be women of all ages and elderly folks, who can benefit from this invention the most.

### DEFINITIONS

As used herein, the "delivery system" comprises a frozen dessert, a collagen peptide (hydrolysate) and one or more bioactive ingredients which are substantially uniformly and completely dispersed and in which degradation of collagen and other bioactives is minimized or eliminated.

As used herein, "collagen" refers to a long-chain of amino acids that builds our skin, connective tissue and bones but cannot be absorbed through our diet. It is made primarily of three amino acids-proline, hydroxyproline and glycine-which gives it unique functional properties different from all other proteins. This also makes it the most important structural protein in the body. Collagen is formed from three very long chains of over 1,000 amino acids twisting into a helix conformation. This tightly twisted helix gives collagen the strength to build our body, but is difficult to break down during digestion and too large to cross the intestinal wall, so in its unhydrolyzed, full-length form, collagen is not an effective oral supplement.

As used herein, "Type I Collagen" refers to a collagen that comprises 90% of skin, hair, nails, organs, bone, ligaments. Type II Collagen applies to cartilage. Type III Collagen applies to fibrous protein in bone, cartilage, dentin, tendon, and other connective tissues. For skin and beauty (i.e. anti-aging), Type I Collagen is considered to be the best. Research in dermatology has demonstrated that when Type I collagen intake is increased, the results are visible in the skin.

As used herein, "collagen peptide" and "collagen hydrolysate" as used interchangeably herein include collagen peptides that are made by breaking down the full-length collagen molecules. They are made of the same amino acids as collagen, but they have different properties. Collagen peptides are more bioavailable - they are better absorbed into the bloodstream because they are much shorter chains of amino acids than collagen and gelatin. Because they're shorter, collagen peptides are more readily broken down into a form that can enter the bloodstream upon digestion.

After absorption, collagen peptides travel throughout the body, repairing, rebuilding and providing energy. Collagen peptides are shuttled to the different tissues where cells will build the peptides into full-length collagen helices to repair our skin, bones and joints, or the cells can use the amino acids directly for energy. Hydrolyzed collagen and collagen peptides are two names for the same product, where full length collagen is broken down into collagen peptides through a process called hydrolysis, so collagen peptides are frequently also referred to as hydrolyzed peptides.

As used herein, the terms "bioactive ingredient", "bioactive agent" and "bioactive" as used interchangeably herein include physiologically or pharmacologically active substances that provide some degree of nutritional or therapeutic benefit when consumed. Non-limiting examples include drugs, botanical extracts, enzymes, hormones, proteins, polypeptides, antigens, nutritional supplements such as fatty acids, amino acids, antioxidants, vitamins, minerals, and other pharmaceutically or therapeutically useful compounds.

As used herein, "frozen dessert" refers to a wide variety of frozen confections including, but not limited to, ice cream, gelato, frozen yogurt, frozen custard, frozen shakes, smoothies, ice milk, sherbet, frozen novelties, frozen dairy confections and frozen non-dairy desserts such as frozen water ices.

As used herein, "nutraceuticals" refers to a food containing health-giving additives and having medicinal benefit.

As used herein, "botanicals" refers to a substance obtained from a plant and used as an additive, especially in gin or cosmetics.

As used herein, "flavoring agent" refers to chemical compounds or molecules such as flavor essences or oils derived from plants, roots, beans, nuts, leaves, flowers, fruits and so forth, equivalent synthetic materials, and mixtures thereof, that are added to flavor a comestible. Flavoring agents are well known in the art. Examples of suitable flavors include, but are not limited to, natural or artificial fruit flavors, such as lemon, orange, banana, grape, lime, apricot, grapefruit, apple, strawberry and cherry, chocolate, pineapple, coffee, vanilla, cocoa, cola, peanut, almond, licorice and cinnamon. The amount of flavoring agent employed is a matter of preference but in general a flavoring agent is used in amounts up to about or at 5%, usually from about or at 0.1% to about or at 1%, by Weight of the composition. The flavoring agents can be used alone or in any combination. Some flavoring agents can be used as masking agents to cover or mask undesirable flavor notes or attributes.

### BACKGROUND OF THE INVENTION

This application and its disclosure generally relate to the field of delivering collagen derivative into a human body.

Various methods and systems of delivering collagen based substances into a human body have been developed over the years. For example, in the beauty and cosmetics industry some forms of collagen are administered to people externally in the form of creams, serums, lotions, etc., which could be quite expensive and somewhat limiting as to how and where these could be applied. In the dietary supplements industry, collagen derivatives are administered to people in the form of pills, powders and mixed drinks. However, a lot of people find these methods of delivery unpalatable, hard to swallow or cumbersome to make. In the food industry collagen derivatives are widely used and administered in the form of undenatured protein powders, gelatins, broths, etc. Given that these forms of collagen have only undergone partial hydrolysis, it has not been fully broken into peptides, so it is not absorbed as well as collagen peptides. The partially hydrolyzed chains in gelatin holds on to a lot of water, so supplementing with gelatin can cause bloating and intestinal discomfort. It's also less versatile than collagen peptides; gelatin will only dissolve in hot water, while collagen peptides will dissolve in both hot and cold liquids; collagen peptides are more bioavailable and digested more quickly than gelatin or broth due to their shorter length.

EP-A1-2 426 138, EP-A1-2 088 157, Liying Li et al: "Effect of Porcine Collagen Peptides on the Rheological and Sensory Properties of Ice Cream", Korean Journal for Food Science of Animal Resources, vol. 35, no. 2, 30 April 2015, and My Nuyen: "Coconut Berry Ice-cream Sandwiches with Collagen Peptides", 13 August 2017, XP002779313 disclose prior art compositions comprising collagen peptides having an antiaging effect.

To eliminate problems associated with prior art methods of delivering and administering collagen derivatives into the human body, the present invention discloses a process for preparing an oral delivery system for collagen peptides, suitable for administration to both human and non-human animals.

### BRIEF DESCRIPTION OF THE FIGURES

The invention is illustrated in the figures of the accompanying drawings in which references denote corresponding parts, and in which:

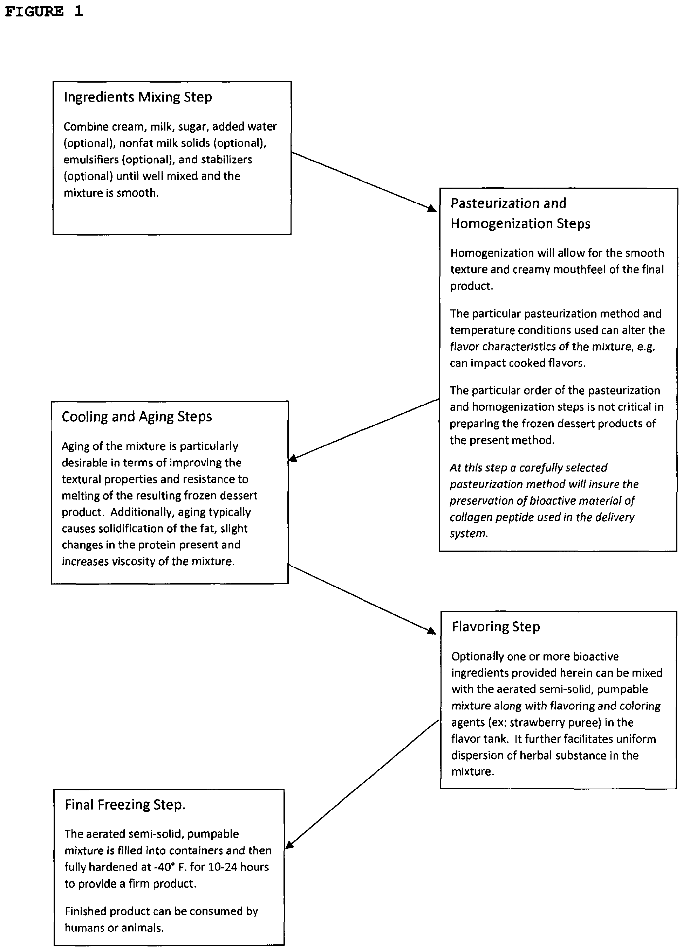

Figure 1 is a schematic diagram of the preparation steps of the delivering system which is designed to deliver collagen peptide into a human body in accordance with the process of the present invention.

### DETAILED DESCRIPTION OF THE INVENTION

### THE DELIVERY SYSTEM

The present invention is defined by the claims.

The present invention discloses a process for preparing an oral delivery system for collagen peptides, suitable for administration to both human and non-human animals, the oral delivery system comprising a frozen dessert and a collagen peptide, the total amount of collagen peptide in the delivery system being less than or equal to 2 0% by weight of the delivery system, the process comprising the following steps:
- adding the collagen peptides to the ingredients of a typical commercial ice cream product;
- passing the mixture through either a single-stage, or a double-stage, homogenizer;
- cooling and aging the homogenized mixture;
- freezing the homogenized mixture by subjecting it to agitation, whipping and aeration to incorporate a selected amount of air, and to avoid the formation of large ice crystals in, or a stratification of, the product; wherein said homogenized mixture is rapidly cooled to a temperature comprised in the range from 0°C (32 °F) to 4,5°C (40°F) and is then held in this temperature range for a period from 1 hour to 12 hours.

The delivery system comprises a frozen dessert, a collagen peptide and one or more bioactive ingredient which are substantially uniformly and completely dispersed and in which degradation of collagen peptide and other bioactives is minimized or eliminated.

The oral delivery system for collagen peptide formulations comprises: 1) a frozen dessert; 2) a collagen peptide; and 3) optionally one or more bioactive ingredients which are substantially uniformly and completely dispersed, and in which degradation of the collagen peptide and other bioactives is minimized or eliminated.

Due to the substantially uniform and complete dispersion of the collagen peptide and other bioactives within the delivery system, the delivery system is suitable for division into subunits. For example, if a single unit of the delivery system is divided into three subunits, each subunit will contain a third of the dose of the original unit. Such division would not be possible with other delivery systems in which the bioactive components are not evenly dispersed.

The delivery system prepared by the process according to the present invention is suitable for administration to both human and non-human animals. One skilled in the art will appreciate that the delivery system can be formulated differently according to the type of animal to which it is to be administered. For example, for administration to an animal such as a cat or a dog, meat or fish-based flavors may be added. For administration to a human, the delivery system may be optionally formulated with addition of flavoring agent.

Minimization or elimination of the degradation of collagen peptide and other bioactives in the delivery system is achieved through the use of low temperatures in the typical ice cream manufacturing, as well as subjecting the collagen peptide ingredient to a special pasteurization process, which ensures that the active compounds are preserved.

In its final form, the delivery system is a firmly frozen product. While in the final freezing step, the air is incorporated to 100% overrun at -5.55°C (22°F) for 25-40 seconds in a continuous freezer to provide an aerated semi-solid, pumpable mixture which is filled into containers and then fully hardened at -40°C (-40°F) for 10-24 hours to provide a firm product. Alternatively, the delivery system is a semi-solid soft serve ice cream or frozen dessert. Soft serve products usually are frozen in a special soft serve freezer, are dispensed by extrusion at carefully chosen subfreezing temperatures and they stand up in a cone or dish upon extrusion. Conventional soft serve products are usually dispensed at an overrun on the order of 40% to 60%. Soft serve products of this character have been known for many years and are available primarily from stores having special freezers that dispense the product for immediate consumption. The soft serve products are usually dispensed at temperatures between - 8.88 °C (16°F) and -4.44°C (24°F). At lower temperatures, the product is generally no longer soft.

The delivery system is especially suited for oral administration due to its palatability and well acceptance as a pleasurable frozen treat. Additionally, due to its highly portable format, the delivery system is simple and convenient to administer and to consume for both humans and other animals.

### COLLAGEN PEPTIDE IN THE DELIVERY SYSTEM

It will be understood that the amount of collagen peptide to be included in the delivery system will be dependent upon the origin of the collagen peptide (animal, marine or botanical origin) that is being administered and also upon whether other bioactives are to be included in the system. In the absence of other bioactives, the total amount of collagen peptide in the delivery system, however, will be less than or equal to 20% by Weight. Collagen peptide and its active compounds are incorporated into the delivery system at levels sufficient to affect the structure or function of the body when taken regularly. Such levels are known in the art of dietary and/or collagen based supplements or can readily be determined by a skilled technician. It is understood that the total daily intake may be based on administration of one unit of the delivery system, or it may be based on administration of more than one unit. The amount of collagen peptide in the final product will thus vary depending on the format of the units and the number to be administered daily. The preferred ratio of ingredients is: 1 serving of frozen dessert to 1 recommended dose of the health promoting collagen peptide. In one embodiment (not claimed), the total amount of collagen peptide in the delivery system is less than or equal to 6% by Weight.

Bioactive ingredients are incorporated into the delivery system at levels sufficient to affect the structure or function of the body when taken regularly. Such levels are known in the art or can readily be determined by a skilled technician. It is understood that the total daily intake may be based on administration of one unit of the delivery system, or it may be based on administration of more than one unit. The amount of bioactive ingredients in the final product will thus vary depending on the format of the units and the number to be administered daily. The preferred ratio of ingredients is: 1 serving of frozen dessert to 1 recommended dose of the desired bioactive ingredient.

Typically, the total amount of collagen peptide including the one or more sources of bioactive ingredient constitute less than about 20% by Weight of the delivery system. For example, in one embodiment (not claimed), where the collagen peptide used is Type I marine collagen. The recommended dose of this peptide is 10g to 15g/day or about 5g per single recommended dose. The single serving of ice cream in the provided example is 85 grams per 4 fl. oz. serving size. Thus the total amount of collagen peptide constitutes less than or equal to 6% by Weight in a single unit (serving) of the delivery system. The calculations are as follows: (100% x 5g) / 85g = 5.88%)

### RECOMMENDED DAILY ALLOWANCE (RDA) OF COLLAGEN PEPTIDE USED IN THE WORKING EXAMPLE

According to Health Canada website (webprod.hc-sc.gc.ca), Minimum 5% of each specific amino acid Recommended Dietary Allowance to a maximum of 10 g hydrolyzed collagen, per day (JC 2012; Benito-Ruiz *et al.* 2009; IOM 2005; Moskowitz 2000). *(Source:* http://webprod.hc-sc.gc.ca/nhpid-bdipsn/atReq.do?atid=hydrolized.collagen*)*.

The collagen peptide is incorporated into the delivery system at levels sufficient to affect the structure or function of the body when taken regularly. In one embodiment (not claimed) where the collagen peptide is Type I marine collagen hydrolysate. The recommended dose of this collagen is about 5g per single recommended dose. In the same embodiment 1 serving of frozen dessert is combined with 1 recommended dose of the collagen peptide which makes 1 unit of delivery system. Hence by consuming one or two units of such delivery system per day, one can functionally accumulate a stimulating dose of collagen in his/her body to promote its' long known anti-aging effects.

Other suitable nutritional supplements include vitamins and minerals that the body is usually not capable of synthesizing and which are necessary for ensuring normal growth and/or daily body maintenance. In the context of the present invention, the vitamins can be hydrosoluble or liposoluble vitamins. Examples includes, but are not limited to, Vitamin A (axerophtol or retinol), Vitamin D, Vitamin E (alpha-tocopherol), Vitamin K, Vitamin B and/or PP (niacinamide or nicotinic acid amide) and Vitamin C (L-ascorbic acid). The dosage of vitamins in the delivery system can be adapted to specific needs. In general, one unit of the delivery system may contain a fraction of the recommended daily amount (RDA) of the desired vitamin. For example, assuming a daily consumption of five units of the delivery system, and following European RDA recommendations, Vitamin A can be used up to 160 pg typically between 70 pg and 90 pg a single unit; Vitamin C up to 12 mg typically between 5 mg and 7 mg a single unit; Vitamin E up to 2 mg typically between 0.8 mg and 1.2 mg a single unit; Vitamin D up to 1 pg typically between 0.4 pg and 0.6 pg a single unit; Vitamin B1 up to 0.28 mg typically between 0.12 mg and 0.15 mg a single unit.

### PROCESS FOR PREPARING THE DELIVERY SYSTEM

### COMMERCIAL MANUFACTURING

Frozen desserts can be made by any commercial manufacturing method known to one skilled in the art. Ice Cream by Arbuckle (2nd edition, 1972 Avi Publishing Co., Westport, Conn., USA) or its various editions defines terminology in relation to the ice cream and related frozen novelty business as well as disclosing compositions, methods of molding, handling procedures, freezing procedures, storage procedures, etc. For example, in a typical commercial ice cream operation, a mixture of cream, milk, sugar, added water (optional), added nonfat milk solids (optional), emulsifiers (optional), and stabilizers (optional) is formed, pasteurized and then passed through either a single, or double-stage, homogenizer. During homogenization, the globules of milkfat that are present in the cream and milk are broken up and dispersed as relatively small fat droplets or particles in a continuous aqueous phase, i.e. an oil-in-water emulsion is formed. During the freezing step, the homogenized mixture is typically subjected to agitation, whipping and aeration to incorporate a selected amount of air (referred to as overrun), and to avoid the formation of large ice crystals in, or a stratification of, the product. Flavoring substances (e.g., vanilla) and optional inclusions are typically added to this homogenized mixture before it is fully hardened to provide a firm ice cream product. Because of the relatively small particle size of the dispersed milkfat due to homogenization, as well as the small particle size of the dispersed ice crystals and air cells formed during freezing, conventional firm ice cream products provide a relatively smooth, creamy mouthfeel.

One skilled in the art will appreciate that molecular interaction between the collagen peptide, the selected frozen dessert and additional bioactive ingredient may affect the physical and bioactive attributes of the final product. The studies has shown that the addition of low molecular-weight collagen peptides was effective in improving the physical and rheological properties of the ice cream. The issue exists with preserving the bioactive material of collagen peptide during the ingredients pasteurization phase, when the peptides are exposed to the extensive heat treatment and could become denatured during the pasteurization process in the commercial ice cream manufacturing. To preserve the high bioactivity of collagen peptide the pasteurization can be carried out by high temperature short-time methods (e.g., at a temperature of at least about or at 79.44°C (175°F) for at least about or at 25 seconds) or vacreation methods (e.g., at a temperature of at least about or at 90°C (194°F) for from about or at 1 second to about or at 3 seconds)

### COMMERCIAL MANUFACTURING METHOD USING COLLAGEN PEPTIDE

In a typical commercial ice cream operation, a mixture of cream, milk, sugar, added water (optional), added nonfat milk solids (optional), emulsifiers (optional), and stabilizers (optional) is formed. The liquid mixture is blended and mixed until the mixture is smooth. The components of the mixture can be combined or added together in any appropriate fashion, and in any order of addition. The fluid mixture is then heated and optionally pasteurized.

Pasteurization can be carried out according to any suitable method that is used in pasteurizing conventional frozen dessert products such as ice cream. See Arbuckle, Ice Cream, (2nd edition, 1972 Avi Publishing Co.) at pages 211-215, which describes the pasteurization of conventional ice cream products. For example, pasteurization can be carried out by batch methods (e.g., at a temperature of at least about or at 68.33°C (155°F), for at least about or at 30 minutes), high temperature short-time methods (e.g., at a temperature of at least about or at 79.44°C (175°F) for at least about or at 25 seconds), vacreation methods (e.g., at a temperature of at least about or at 90°C (194°F) for from about or at 1 second to about or at 3 seconds), and ultrahigh temperature methods (e.g., at a temperature of from about or at 98.88°C (210°F) to about or at 129.44°C (265°F) for from about or at 2 seconds to about or at 40 seconds). The particular pasteurization method and temperature conditions used can alter the flavor characteristics of the mixture, e.g., can impart cooked flavors. However, to preserve the maximum bioactivity of collagen peptide the pasteurization should be carried out by a method with a shortest heat exposure time. Such as, high temperature short-time methods (e.g., at a temperature of at least about or at 79.44°C (175°F) for at least about or at 25 seconds) or vacreation methods (e.g., at a temperature of at least about or at 90°C (194°F) for from about or at 1 second to about or at 3 seconds)

This heated, fluid mixture is then subjected to a homogenization step. Homogenization is usually accomplished by forcing this fluid mixture through the small orifice of a homogenizer (or orifices in the case of a two-stage homogenizer), using a positive displacement plunger pump to furnish the appropriate pressure. This orifice includes a valve and seat in which the two adjacent surfaces are parallel and lap smooth and is surrounded by an impact ring against which the fluid mixture of ingredients impinges as it leaves the valve. The breakup and size reduction of the fat droplets is caused by the shear forces that occur as a thin stream of the fluid mixture travels at a high velocity between the closely adjacent surfaces of the valve and the seat, and then by the shattering effect that occurs as the thin stream impinges on the impact ring upon leaving the valve. Size reduction of the fat droplets is also caused by cavitation effects. Cavitation is caused by the sudden release of pressure as the thin stream leaves the valve, which momentarily lowers the vapor pressure of the fluid mixture to a point where vapor pockets are formed. The fat droplets bounce back and forth inside these vapor bubbles and are shattered by impacts against the bubble walls, thus causing further size reduction.

The homogenization of this fluid mixture can be carried out by passing the heated fluid mixture through either a one-stage or a two-stage homogenizer. See Arbuckle, Ice Cream, (1977 Avi Publishing Co.), pp. 216-218, for suitable one-stage and two-stage homogenizers, including those manufactured and sold by Gaulin and Cherry-Burrell Corp. In the case of one-stage homogenizers, suitable operating pressures (measured in pounds per square inch or psi) can be in the range of from about or at 800 to about or at 3000 psi, usually from about or at 1500 to about or at 2000 psi. In the case of two-stage homogenizers, the first stage can be operated at a pressure of from about or at 800 psi to about or at 3000 psi, or from about or at 1500 psi to about or at 2000 psi, while the second stage is operated at a pressure of from about or at 500 psi to about or at 1000 psi.

The particular order of the pasteurization and homogenization steps is not critical in preparing the frozen dessert products of the present method. For example, the fluid mixture can be homogenized, and then pasteurized, or can be pasteurized and then homogenized.

The homogenized pasteurized mixture is rapidly cooled to a temperature of 4.5 °C 40° F) or less, and typically to a temperature in the range of from 0° (32° F) to 4.5°C (40° F). The cooled mixture is then held in this temperature range for a period of from 1 hour to 12 hours, or for from 1 hour to about or at 2 hours, to age the mixture. Aging typically causes the following effects to occur in the mixture: (1) solidification of the fat; (2) slight changes in the protein present; and (3) increases in the viscosity of the mixture. Aging of the mixture is particularly desirable in terms of improving the textural properties and resistance to melting of the resulting frozen dessert product, as well as ease in incorporating air during subsequent freezing. See Arbuckle, Ice Cream, (2nd edition, 1972 Avi Publishing Co.), at page 222.

This homogenized pasteurized mixture optionally can be packaged at this point as a liquid ice cream mix or base, for example for use by restaurants, food suppliers or for consumer use in home freezers.

The homogenized pasteurized mixture, is subjected to a freezing step to partially freeze or solidify the mixture. The partial freezing of this homogenized pasteurized mixture can be carried out by any standard freezing method used in the preparation of conventional frozen dessert products such as ice cream. See Arbuckle, Ice Cream, (2'd edition, 1972 Avi Publishing Co.), pages 239-266. For example, the homogenized pasteurized mixture can be partially frozen or solidified by using a batch freezer, continuous freezer, low temperature continuous freezer, a soft serve-type freezer, or a counter-type freezer. The particular temperature and time conditions for carrying out this partial freezing step can vary greatly depending upon the type of freezer used, and can be determined empirically.

For example, the homogenized pasteurized mixtures of the present method can be partially frozen at temperatures in the range of from about or at -9.44°C (15°F) to about or at -2.22 (28°F) over a period of from about or at 20 seconds (e.g., continuous or low temperature continuous freezer) to about or at 10 minutes (e.g., batch or counter freezer). During partial freezing, it is often desirable to agitate, aerate and/or whip the mixture to incorporate air to provide a selected amount of overrun. The particular amount of overrun obtained can be any level appropriate for conventional frozen dessert products, in particular ice cream products, and can be determined empirically by one skilled in the art.

For example, a mixture of ingredients can be formed by adding fluid milk, cream, the low calorie sugar substitute provided herein, water, non-fat dry milk solids and egg yolks to a mix tank in the order indicated or in any order or combination. The contents of the mix tank can be mixed together and heated to a temperature of from 62.77°C (145°F) to 65.55°C (150°F), and then can be passed through a two-stage homogenizer operated at a pressure of 1500 psi in the first stage and 500 psi in the second stage. This homogenized mixture then can be pasteurized at 79.44°C (175°F) for 25 seconds. This homogenized pasteurized mixture then can be cooled to a temperature of approximately 4.44°C (40°F), and then can be aged at this cooler temperature for 1 to 2 hours. Flavor, such as vanilla, and optionally coloring then can be added.

The flavored and optionally colored mixture then can be chilled while incorporating air to 100% overrun at about or at - 5.55°C (22°F) for 25-40 seconds in a continuous freezer to provide an aerated semi-solid, pumpable mixture which is filled into containers and then fully hardened at -40°C (-40°F) for 10-24 hours to provide a firm product.

The mix can also be used to make soft serve ice creams or soft serve ice milks. Soft serve products usually are frozen in a special soft serve freezer, are dispensed by extrusion at carefully chosen subfreezing temperatures and they stand up in a cone or dish upon extrusion. Conventional soft serve products are usually dispensed at an overrun on the order of 40% to 60%. Soft serve products of this character have been known for many years and are available primarily from stores having special freezers that dispense the product for immediate consumption. The soft serve products are usually dispensed at temperatures between -8.88°C (16°F) and -4.44°C (24°F). At lower temperatures, the product is generally no longer soft. There is considerable published art on the subject of soft frozen desserts, particularly ice cream. A pertinent text is Ice Cream, Second Edition by W. S. Arbuckle (1972, Avi Publishing Company, Inc., Westport, Conn., pp. 278-291).

### TESTING THE DELIVERY SYSTEM

Once the delivery system has been prepared, it may be tested to ensure that it meets the desired criteria, ie that the collagen peptide and other bioactives are substantially uniformly dispersed, that degradation of these compounds during the preparation of the delivery system is below 15%.

For example, dispersion of the product can be determined by dividing a single unit of the final delivery system into several subunits and analyzing the content of collagen peptide in each subunit. Collagen peptide levels can readily be measured by standard analytical techniques such as high performance liquid chromatography (HPLC). If the % by Weight of collagen peptide in each subunit is similar, then the collagen peptide is said to be substantially uniformly dispersed throughout the product. One skilled in the art will appreciate that the % by Weight need not be identical for each subunit to indicate uniform dispersion.

In addition, the delivery system may undergo testing to evaluate such factors as the microbial content of the product and the shelf-life of the product. Such quality control testing is standard in the art and can be conducted using known methods.

For example, microbial analysis of the delivery system can be conducted using techniques approved by the appropriate regulatory board, such as those described in "The Compendium of Analytical Methods: HPB Methods for the Microbiological Analysis of Foods" issued by the Health Products and Food Branch of Health Canada. Shelf life is typically evaluated using accelerated shelf life tests in which the stability of the system and the degradation of the collagen peptide and bioactives contained therein is analyzed under conditions that are known to accelerate the degradation of food products and can be correlated to the stability of the product under normal storage conditions.

### FORMAT OF THE DELIVERY SYSTEM

The present invention contemplates various formats for the delivery system. For example, the delivery system may be in the form of firmly frozen dessert or semi-solid frozen dessert.

### METHOD OF ADMINISTRATION

The organoleptic properties of the delivery system prepared by the process of the present invention ensure that it is easy to take and/or to administer. The delivery system is formulated for administration to humans and thus contains a frozen dessert with flavors that would appeal to humans, such as fruit-based flavors.

In another embodiment, the delivery system may be formulated for administration to a non-human animal. In this embodiment the non-human animal is a domestic animal, such as a dog or a cat.

To gain a better understanding of the invention described herein, the following example is set forth. It should be understood that this example is for illustrative purposes only. Therefore, they should not limit the scope of this invention in any way.

### EXAMPLES

### EXAMPLARY FORMULATION (not part of the invention)

The example describes a possible formulation for the oral delivery system with a collagen peptide and bioactive ingredients suitable for muscle and skin enhancement such as whey protein and vitamins A and D3.

For a working Example 1, defined in Nutrition Facts Table 1, a mixture of ingredients were formed by adding cream, skim milk, sugar, whey, egg yolks, xanthan gum, locust bean gum, guar gum cream and collagen peptide, to a mix tank without any specific order or combination. The contents of the mix tank were thoroughly mixed together and pasteurized, where the mixture was heated at a temperature of 79.44°C (175°F) for approximately 25 seconds. Then the liquid mixture was passed through a one-stage homogenizer operated at a pressure of 2000 psi. This pasteurized and homogenized mixture then was cooled to a temperature of approximately 4.44°C (40°F), and aged at this cooler temperature for 24 hours. After the aging period, the mixture was pumped into the flavoring tank where natural coconut extract and vitamins A and D3 were added to the mix. While in the flavoring tank the mixture was thoroughly mixed again and pumped into a continuous freezer where it was chilled while incorporating air to 50% overrun at about -5.55°C (22°F) for about 40 seconds to provide an aerated semi-solid, pumpable mixture which was filled into containers and then fully hardened at -40°C (-40°F) for 24 hours to provide a firm product.

### EXAMPLE 1

Example of an oral delivery system with Type I marine collagen peptide and other bioactive ingredients such as whey protein and vitamins A and D3, where the delivery system is presented in the form of premium ice cream with 12% fat content.

### Shelf-Life Determination

A shelf life can be obtained through a conventional method of product sampling and observation. The samples are subjected to monthly sampling and physical observation for texture, appearance and quality. Based on these observations, the delivery system is shown to have a stable shelf life of at least one year from the date of manufacture.

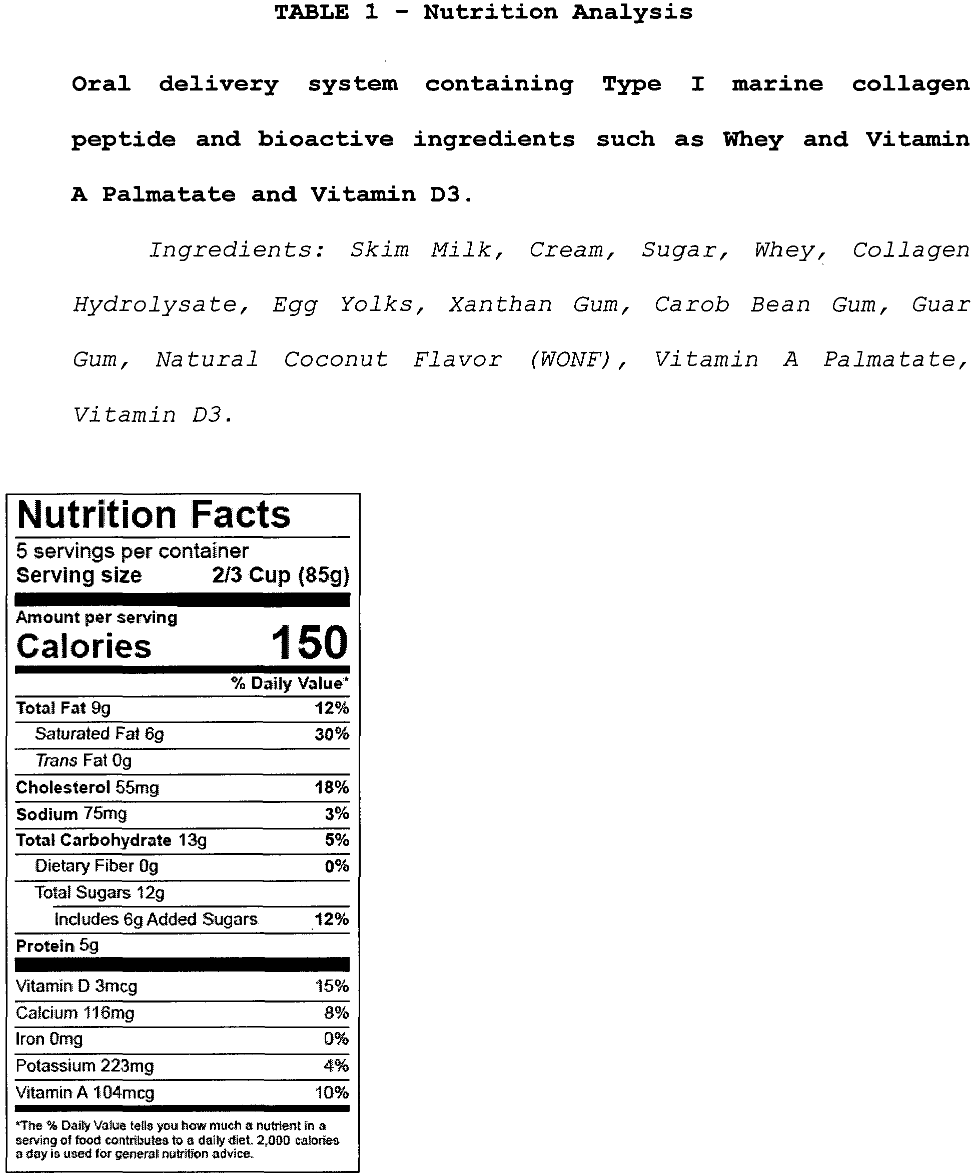

## Claims

1. Process for preparing an oral delivery system for collagen peptides, suitable for administration to both human and non-human animals, the oral delivery system comprising a frozen dessert and a collagen peptide, the total amount of collagen peptide in the delivery system being less than or equal to 20% by weight of the delivery system, the process comprising the following steps:
• adding the collagen peptides to the ingredients of a typical commercial ice cream product;
• passing the mixture through either a single-stage, or a double-stage, homogenizer;
• cooling and aging the homogenized mixture;
• freezing the homogenized mixture by subjecting it to agitation, whipping and aeration to incorporate a selected amount of air, and to avoid the formation of large ice crystals in, or a stratification of, the product;
wherein said homogenized mixture is rapidly cooled to a temperature comprised in the range from 0°C (32°F) to 4,5°C (40°F) and is then held in this temperature range for a period from 1 hour to 12 hours.

2. Process according to claim 1, **characterized in that** it further comprises one or more bioactive ingredients which are substantially uniformly and completely dispersed.

3. Process according to claim 2, **characterized in that** the ratio of ingredients is 1 serving of frozen dessert to 1 dose of the collagen peptide, the total amount of collagen peptide in the delivery system being less than or equal to 6% by weight of the delivery system.

4. Process according to any one of claims 1 to 3, **characterized in that** said other suitable bioactive ingredients include vitamins and minerals that the body is usually not capable of synthesizing and which are necessary for ensuring normal growth and/or daily body maintenance, said vitamins being hydro-soluble or liposoluble.

5. Process according to claim 4, **characterized in that** said vitamins include one or more of the following:
• vitamin A (axerophtol or retinol);
• Vitamin D;
• Vitamin E (alpha-tocopherol);
• Vitamin K;
• Vitamin B and/or PP (niacinamide or nicotinic acid amide);
• Vitamin C (L-ascorbic acid).

6. Process according to any one of claims 1 to 5, **characterized in that** it comprises a mixture of the following ingredients:
• cream, skim milk, sugar, whey, egg yolks, xanthan gum, locust bean gum, guar gum cream;
• collagen peptide;
• coconut extract;
• vitamins A and D3.

7. Process according to claim 1, **characterized in that** said homogenized mixture is rapidly cooled to a temperature of 4,5°C (40°F) and is then held in this temperature range for a period from 1 hour to 2 hours.

8. Process according to claim 1, **characterized in that** one or more bioactive ingredients are added to said mixture before it is fully hardened to provide a firm ice-cream product, and **in that** flavoring substances and optional inclusions are added to said mixture before it is fully hardened to provide a firm ice-cream product.

9. Process according to at least one of claims 1 or 7 to 8, **characterized in that** said mixture is pasteurized before it is fully hardened to provide a firm ice-cream product.

10. Process according to claim 9, **characterized in that** said pasteurization is carried out at a temperature of at least 79,4°C (175°F) for at least 25 seconds.

11. Process according to claim 9, **characterized in that** said pasteurization is carried out by a vacreation method, at a temperature of at least 90°C (194°F) for 1 second to 3 seconds.

12. Process according to any one of claims 1 or 7 to 10, **characterized in that** the delivery system is in the form of firmly frozen dessert or semi-solid frozen dessert.

13. Process for preparing an oral delivery system for collagen peptides according to any one of claim 1 or 7 to 12, **characterized in that** it comprises the following steps:
• forming a mixture of ingredients by adding cream, skim milk, sugar, whey, egg yolks, xanthan gum, locust bean gum, guar gum cream and collagen peptide, to a mixing tank;
• thoroughly mixing together said ingredients;
• pasteurizing said mixture at a temperature of 79,4°C (175°F) for approximately 25 seconds;
• passing the pasteurized mixture through a one-stage homogenizer operated at a pressure of 2000 psi;
• cooling said pasteurized and homogenized mixture to a temperature of approximately 4,5°C (40°F);
• aging the mixture maintaining said temperature of 4,5°C (40°F) for 24 hours;
• pumping the mixture into a flavoring tank where natural coconut extract and vitamins A and D3 are added;
• while in the flavoring tank, thoroughly mixing again and pumping the mixture into a continuous freezer, the mixture being chilled while incorporating air to 50% overrun at -5,55°C (22°F) for 40 seconds to provide an aerated semi-solid, pumpable mixture;
• filling said pumpable mixture into containers and then fully hardening said mixture at -4,5°C (40°F) for 24 hours to provide a firm product.

## Patentansprüche

1. Verfahren für die Zubereitung eines oralen Verabreichungssystems für Kollagenpeptide, die sowohl für die Verabreichung an Menschen als auch an Tiere geeignet sind, das Verabreichungssystem enthält ein gefrorenes Dessert und ein Kollagenpeptid, die Gesamtmenge des Kollagenpeptides im Verabreichungssystem ist geringer als oder entspricht 20 % des Gewichtes des Verabreichungssystems, das Verfahren enthält die folgenden Phasen:
• Hinzufügung der Kollagenpeptide zu den Zutaten eines typischen Handelseises;
• Durchführung der Mischung durch einen Einstufen- oder Zweistufen-Homogenisator;
• Abkühlung und Alterung der homogenisierten Mischung;
• Einfrieren der homogenisierten Mischung und Schütteln, Schaumschlagen und Durchlüften, um eine ausgewählte Luftmenge beizumengen und die Bildung von Eiskristallen oder einer Schichtung des Produkts zu vermeiden;
wobei die genannte homogenisierte Mischung schnell auf eine Temperatur im Bereich von 0 °C (32 °F) bis 4,5 °C (40 °F) abgekühlt und dann für einen Zeitraum von 1 bis 12 Stunden in diesem Temperaturbereich gehalten wird.

2. Verfahren gemäß Patentanspruch 1, das **dadurch gekennzeichnet ist, dass** es außerdem eine oder mehrere bioaktive Zutaten enthält, die grundlegend gleichmäßig und vollständig verteilt werden.

3. Verfahren gemäß Patentanspruch 2, das **dadurch gekennzeichnet ist, dass** das Verhältnis der Zutaten 1 Dosis gefrorenes Dessert zu 1 Dosis Kollagenpeptiden ist, die Gesamtmenge des Kollagenpeptides im Verabreichungssystem ist geringer als oder entspricht 6 % des Gewichtes des Verabreichungssystems.

4. Verfahren gemäß einem beliebigen der Patentansprüche von 1 bis 3, das **dadurch gekennzeichnet ist, dass** die genannten anderen bioaktiven Zutaten Vitamine und Mineralien enthalten, die der Körper normalerweise nicht synthetisieren kann und die notwendig sind, um ein normales Wachstum und/oder eine normale tägliche Erhaltung des Körpers zu garantieren, die genannten Vitamine sind wasserlöslich oder fettlöslich.

5. Verfahren gemäß Patentspruch 4, das **dadurch gekennzeichnet ist, dass** die genannten Vitamine eines oder mehrere der Folgenden enthalten:
• Vitamin A (Axerophthol oder Retinol);
• Vitamin D;
• Vitamin E (Alpha-Tocopherol);
• Vitamin K;
• Vitamin B und oder PP (Niacinamid oder Nicotinsäureamid);
• Vitamin C (L-Ascorbinsäure).

6. Verfahren gemäß einem beliebigen der Patentansprüche von 1 bis 5, das **dadurch gekennzeichnet ist, dass** es eine Mischung der folgenden Zutaten enthält:
• Creme, Magermilch, Zucker, Gerste, Eigelb, Xanthangummi, Johannesbrotkernmehl, Guarkenmehl Creme;
• Kollagenpeptiden;
• Kokusnussextrakt;
• Vitamin A und D3.

7. Verfahren gemäß Patentanspruch 1, das **dadurch gekennzeichnet ist, dass** die genannte homogenisierte Mischung schnell auf eine Temperatur im Bereich von 4,5 °C (40 °F) abgekühlt und dann für einen Zeitraum von 1 bis 2 Stunden in diesem Temperaturbereich gehalten wird.

8. Verfahren gemäß Patentanspruch 1, das **dadurch gekennzeichnet ist, dass** eine oder mehrere der bioaktiven Zutaten zu dieser Mischung hinzugefügt werden, bevor sie vollkommen gehärtet wird, um ein festes Eisprodukt zu bieten, und dadurch, dass die Aromastoffe und die optionalen Magerungspartikel zu dieser Mischung hinzugefügt werden, bevor sie vollkommen gehärtet ist, um ein festes Eisprodukt zu bieten.

9. Verfahren gemäß mindestens einem der Patentansprüche 1 oder von 7 bis 8, das **dadurch gekennzeichnet ist, dass** die genannte Mischung pasteurisiert wird, bevor sie vollkommen gehärtet wird, um ein festes Eisprodukt zu bieten.

10. Verfahren gemäß Patentanspruch 9, das **dadurch gekennzeichnet ist, dass** die genannte Pasteurisierung mindestens 25 Sekunden lang bei einer Temperatur von mindestens 79,4 °C (175 °F) ausgeführt wird.

11. Verfahren gemäß Patentanspruch 9, das **dadurch gekennzeichnet ist, dass** die genannte Pasteurisierung durch eine Niederdruckpasteurisierungsmethode für einen Zeitraum von 1 bis 3 Sekunden bei einer Temperatur von mindestens 90 °C (194 °F) ausgeführt wird.

12. Verfahren gemäß einem beliebigen der Patentansprüche 1 oder von 7 bis 10, das **dadurch gekennzeichnet ist, dass** das Verabreichungssystem die Form eines festen gefrorenen Desserts oder eines halbfesten gefrorenen Desserts hat.

13. Verfahren für die Zubereitung eines oralen Verabreichungssystems für Kollagenpeptide gemäß einem beliebigen der Patentansprüche 1 oder von 7 bis 12, das **dadurch gekennzeichnet ist, dass** es folgende Phasen enthält:
• Bildung einer Mischung von Zutaten mit Hinzufügung von Creme, Magermilch, Zucker, Gerste, Eigelb, Xanthangummi, Johannesbrotkernmehl, Guarkenmehl Creme und Kollagenpeptiden in einer Mischmaschine;
• Gründliches Mischen der genannten Zutaten;
• Pasteurisieren der genannten Mischung für ca. 25 Sekunden bei einer Temperatur von 79,4 °C (175 °F);
• Durchführung der pasteurisierten Mischung durch einen Einstufen-Homogenisator, der mit einem Druck von 2000 psi aktiviert wird;
• Abkühlen der genannten pasteurisierten und homogenisierten Mischung bei einer Temperatur von ca. 4,5 °C (40 °F);
• Altern der Mischung mit Beibehaltung der genannten Temperatur bei 4,5 °C (40 °F) für 24 Stunden;
• Pumpen der Mischung in eine Aromatisierungsmaschine, wo natürlicher Kokusnussextrakt und Vitamin A und D3 hinzugefügt werden;
• Während sich die Mischung in der Aromatisierungsmaschine befindet, gründlich erneut mischen, und die Mischung in einen Freezer pumpen, die Mischung wird abgekühlt, während überschüssige Luft zu 50 % bei -5,55 °C (22 °F) 40 Sekunden lang beigemischt wird, um eine durchlüftete, halbfeste, pumpfähige Mischung zu bieten;
• Einfüllen der genannten pumpfähigen Mischung in Behälter und dann vollständige 24 Stunden lange Härtung der genannten Mischung bei -4,5 °C (40 °F), um ein festes Produkt zu bieten.

## Revendications

1. Processus pour préparer un système d'administration orale pour peptides de collagène, approprié au traitement aussi bien d'êtres humains que d'animaux ; le système d'administration comprend un dessert congelé et un peptide de collagène, la quantité totale de peptide de collagène dans le système d'administration est inférieur ou égal à 20% en poids du système d'administration ; le processus comprend les phases suivantes :
• ajouter les peptides de collagène aux ingrédients d'un produit typique de glace commerciale ;
• faire passer le mélange à travers un homogénéisateur à stade simple ou double ;
• refroidir et vieillir le mélange homogénéisé ;
• congeler le mélange homogénéisé en le soumettant à système d'agitation à l'air et une agitation mécanique au fouet pour incorporer la quantité d'air voulue et éviter la formation de cristaux de glace dans le produit ou une stratification de ce dernier ;
où le mélange homogénéisé est refroidi rapidement à une température comprise entre 0°C (32°F) et 4,5°C (40°F) et est maintenu dans cette plage de températures pendant un intervalle allant de 1 à 12 heures.

2. Processus, selon la revendication 1, **caractérisé en ce qu'**il comprend, en outre, un ou plusieurs ingrédients bioactifs qui sont dispersés de manière substantiellement uniforme et complète.

3. Processus, selon la revendication 2, **caractérisé en ce que** le rapport des ingrédients est 1 dose de dessert congelé pour 1 dose de peptide de collagène ; la quantité totale du peptide de collagène dans le système d'administration est inférieure ou égale à 6% en poids du système d'administration.

4. Processus, selon l'une des revendications de 1 à 3, **caractérisé en ce que** les autres ingrédients bioactifs appropriés comprennent des vitamines et des minéraux que le corps n'est généralement pas en mesure de synthétiser mais qui sont nécessaires pour garantir une croissance normale et/ou un maintien journalier du corps ; ces vitamines sont hydrosolubles ou liposolubles.

5. Processus, selon la revendication 4, **caractérisé en ce que** les vitamines comprennent une ou plusieurs des vitamines suivantes :
• vitamine A (rétinol) ;
• vitamine D ;
• vitamine E (alpha-tocophérol) ;
• vitamine K ;
• vitamine B et/ou PP (niacinamide ou amide d'acide nicotique) ;
• vitamine C (acide L-ascorbique).

6. Processus, selon l'une des revendications de 1 à 5, **caractérisé en ce qu'**il comprend un mélange des ingrédients suivants :
• crème, lait écrémé, sucre, orge, jaunes d'œufs, gomme xanthane, gomme de caroube, crème de gomme de Guar ;
• peptide de collagène ;
• extrait de coco ;
• vitamines A et D3.

7. Processus, selon la revendication 1, **caractérisé en ce que** le mélange homogénéisé est refroidi rapidement à une température de 4,5°C (40°F) et est maintenu dans cette plage de températures pendant un intervalle allant de 1 à 2 heures.

8. Processus, selon la revendication 1, **caractérisé en ce qu'**un ou plusieurs ingrédients bioactifs sont ajoutés au mélange avant qu'il durcisse complètement pour fournir une glace ferme ; et **en ce que** les substances aromatiques et les ajouts optionnels sont additionnés au mélange avant qu'il ne durcisse complètement pour fournir une glace ferme.

9. Processus, au moins selon la revendication 1 ou l'une des revendications de 7 à 8, **caractérisé en ce que** le mélange est pasteurisé avant de durcir complètement pour fournir une glace ferme.

10. Processus, selon la revendication 9, **caractérisé en ce que** la pasteurisation est exécutée à une température non inférieure à 79,4°C (175°F) pendant au moins 25 secondes.

11. Processus, selon la revendication 9, **caractérisé en ce que** la pasteurisation est exécutée à travers un procédé de vacréation à une température non inférieure à 90°C (194°F) pendant un intervalle allant de 1 à 3 secondes.

12. Processus, selon la revendication 1 ou l'une des revendications de 7 à 10, **caractérisé en ce que** le système d'administration est sous forme de dessert congelé ferme ou de dessert congelé semi-solide.

13. Processus pour préparer un système d'administration orale pour peptides de collagène, selon la revendication 1 ou l'une des revendications de 7 à 12, **caractérisé en ce qu'**il comprend les phases suivantes :
• former un mélange d'ingrédients en ajoutant de la crème, du lait écrémé, du sucre, de l'orge, des jaunes d'œufs, de la gomme xanthane, de la gomme de caroube, de la crème de gomme de Guar et du peptide de collagène, dans un bac de mélange ;
• bien mélanger tous les ingrédients ;
• pasteuriser le mélange à une température de 79,4°C (175°F) pendant environ 25 secondes ;
• faire passer le mélange pasteurisé à travers un homogénéisateur à stade simple activé à une pression de 2000 psi ;
• refroidir le mélange pasteurisé et homogénéisé à une température d'environ 4,5°C (40°F) ;
• faire vieillir le mélange en maintenant la température sur 4,5°C (40°F) pendant 24 heures ;
• pomper le mélange dans un bac d'aromatisation où l'on ajoute l'extrait de coco naturel et les vitamines A et D3 ;
• alors que le mélange se trouve encore dans le bac d'aromatisation, bien mélanger à nouveau et pomper le mélange dans un congélateur continu ; le mélange refroidit alors que l'on y incorpore de l'air à 50% d'overrun à -5,55°C (22°F) pendant 40 secondes pour fournir un mélange pompable semi-solide aéré ;
• mettre le mélange pompable dans des récipients puis le faire durcir complètement à -4,5°C (40°F) pendant 24 heures pour obtenir un produit ferme.
